# FASCICULE DE BREVET EUROPEEN

(11) **EP 1 035 110 B1**
(45) Date de publication et mention de la délivrance du brevet: **15.01.2003**
(21) Numéro de dépôt: 00400479.2
(22) Date de dépôt: 22.02.2000
(51) Int. Cl.: C07C 237/06, C07C 237/04, C07D 295/18, C07D 207/26, C07D 401/04, C07D 211/58, A61K 7/48

(54) **Dérivés de benzylaminodiacétamides, compositions les comprenant, procédé de préparation et utilisations**
Benzylaminodiacetamidderivate, Zusammensetzungen, die diese enthalten, Verfahren zu ihrer Herstellung und Anwendungen
Benzylaminodiacetamide derivatives, compositions containing the same, process for their preparation and uses

(30) Priorité: 11.03.1999 FR 9903005
(43) Date de publication de la demande: 13.09.2000
(73) Titulaire: L'OREAL, 75008 Paris (FR)
(72) Inventeur: Galey, Jean-Baptiste, 93600 Aulnay S/Bois (FR); Dalko, Maria, 91100 Gif/S/Yvette (FR)
(74) Mandataire: Dodin, Catherine

(56) Documents cités:
- EP-A- 0 820 763
- EP-A- 0 864 563
- EP-A- 0 869 115
- FR-A- 2 728 793
- CHENG ET AL JOURNAL OF MOLECULAR CATALYSIS vol. 113, 1996, pages 379 - 391

## Description

La présente invention a trait à de nouveaux composés du type benzylaminodiacétamides et leurs dérivés, ainsi qu'à leur utilisation notamment en cosmétique. L'invention concerne également des compositions cosmétiques, en particulier dermatologiques, comprenant lesdits composés.

Dans le domaine des désordres cutanés, il est connu que certaines peaux sont plus sensibles que d'autres. Toutefois, les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.
Des tests ont été mis au point pour cerner les peaux sensibles, par exemple des tests à l'acide lactique et au DMSO qui sont connus pour être des substances irritantes : voir par exemple l'article de K. Lammintausta et coll., Dermatoses, 1988, 36, pages 45-49; et l'article de T. Agner et J. Serup, Clinical and Expérimental Dermatology, 1989, 14, pages 214-217.
Du fait de la méconnaissance des caractéristiques des peaux sensibles, il était jusqu'à présent très difficile voire impossible de les traiter. En fait, on les traitait indirectement, par exemple en limitant dans les compositions cosmétiques l'emploi de produits à caractère irritant tels que les tensioactifs, les conservateurs ou les parfums ainsi que l'emploi de certains actifs cosmétiques.
Or, il a été possible de déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements. Les symptômes liés à la peau peuvent être également des manifestations microvasculaires du tissu cutané comme les érythèmes.
D'autre part, une peau sensible n'est pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.
Les peaux sensibles peuvent être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.
Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont également associés à une peau hyperséborrhéique ou acnéique avec ou sans dartres, et à un érythème.
Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs; eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.
Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.
Pour déterminer si une peau est sensible ou non, il a été mis au point un test. En effet, après avoir effectué un grand nombre de tests dans le but de définir une peau sensible, il a été trouvé de manière surprenante qu'il existait un lien entre les personnes à peau sensible et celles qui réagissaient à une application topique de capsaïcine.
Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.
Les signes cliniques de la peau sensible sont essentiellement subjectifs : picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Il est connu, par le document FR2728793, d'utiliser un antagoniste d'histamine, un antagoniste d'interleukine 1 et/ou un antagoniste de TNF-alpha dans une composition cosmétique ou pharmaceutique, pour traiter les peaux sensibles.

Il est également connu par le document EP869115 des dérivés d'acides acides qui peuvent être employés pour inhiber l'oxygène actif.

Il est encore connu, par le document EP864563, d'employer des esters d'acides aminés hydroxylés et N-acylés pour renforcer la fonction-barrière de la peau.

Il est encore connu, par le document EP820763, d'utiliser des dérivés de l'acide N-N'-dibenzyléthylènediamine N,N'-diacétique, comme agent dépigmentants de la peau.

Après de nombreuses recherches, la demanderesse a mis en évidence de nouveaux composés appartenant à la famille des benzylaminodiacétamides, qui possèdent de bonnes propriétés anti-irritantes et apaisantes. Notamment, ces composés peuvent permettre d'éviter l'irritation cutanée et/ou les sensations dysesthésiques et/ou les prurits de la peau et/ou des muqueuses et/ou l'érythème et/ou les dartres et/ou les sensations d'échauffement, notamment dans une composition destinée à un usage topique, en particulier dans une composition destinée à traiter les peaux sensibles.

La présente invention a donc pour objet une composition comprenant, dans un milieu physiologiquement acceptable, au moins un composé répondant à la formule (I) décrite ci-après.

Certains composés de formule I dans lesquels R¹ = R² = H ont été décrits dans Cheng et al. Journal of Molecular Catalysis, 113 (1996), 379-391.

L'invention concerne également un composé répondant à la formule (la) décrite ci-après.
Un autre objet de l'invention est un procédé de préparation d'un composé de formule (Ia) dans lequel on fait réagir un diacide avec un agent déshydratant de manière à obtenir un anhydride intermédiaire permettant le couplage avec une première amine ou un premier alcool, puis, dans une deuxième étape, on ajoute un agent de couplage, permettant le couplage dudit diacide avec une deuxième amine ou un deuxième alcool.
Encore un autre objet est l'utilisation d'au moins un composé de formule (I) dans une composition cosmétique comprenant par ailleurs un produit à effet irritant, pour atténuer, voire supprimer ledit effet irritant.
Un autre objet de l'invention est l'utilisation d'au moins un composé de formule (I) dans une composition cosmétique ou pour la préparation d'une composition physiologiquement acceptable, pour prévenir et/ou traiter les désordres cutanés, et notamment les irritations cutanées, les dartres, les rougeurs, les sensations dysesthésiques, les sensations d'échauffement et/ou les prurits de la peau et/ou des muqueuses.
Un autre objet de l'invention est un procédé de traitement cosmétique, caractérisé par le fait que l'on applique sur la peau, sur les cheveux et/ou sur les muqueuses, une composition cosmétique telle que définie ci-dessus.

De plus, on a constaté que, de façon avantageuse, les composés de formule (I) peuvent être associés à des produits à effet irritant utilisés couramment, notamment dans le domaine cosmétique, notamment certains actifs cosmétiques. La présence d'au moins un composé de formule (I) dans une composition comprenant un tel produit ayant un effet irritant permet d'atténuer fortement, voire de supprimer cet effet irritant. Cela permet en outre d'augmenter la quantité dudit produit à effet irritant par rapport à la quantité normalement utilisée, en vue d'une efficacité améliorée.

Les composés selon l'invention répondent à la formule générale (I) suivante : 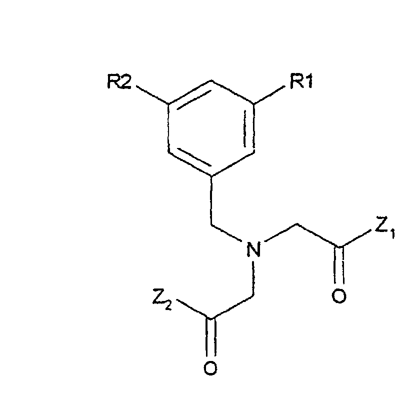 dans laquelle :
- R₁ et R₂, identiques ou différents sont choisis parmi l'hydrogène ou les radicaux CH₃, CF₃, OCH₃ et OH,
- Z₁ est choisi parmi les radicaux NX₁X₂ et OX₁, et Z₂ est choisi parmi les radicaux NX₃X₄ et OX₃, dans lesquels :
- X₁ et X₃, identiques ou différents, représentent :
   - un radical alkyle en C₁-C₁₂, éventuellement substitué par un ou plusieurs groupements OH, NH₂, SH, CN, CF₃, halogène, COOH, CONHR', COOR', OR', SR' avec R' représentant un alkyle en C₁-C₄; ou éventuellement substitué par un ou plusieurs cycles en C₃-C₇ aliphatiques ou aromatiques, éventuellement hétérocycliques; ou
   - un radical aryle, éventuellement substitué par un ou plusieurs groupements OH, NH₂, SH, CN, CF₃, halogène, COOH, CONHR', COOR', OR', SR' avec R' représentant un alkyle en C₁-C₄; ou éventuellement substitué par un ou plusieurs cycles en C₃-C₇ aliphatiques ou aromatiques, éventuellement hétérocycliques; et
- X₂ et X₄, identiques ou différents, représentent un atome d'hydrogène ou forment un cycle à 5 ou 6 chaînons avec X₁ et X₃ respectivement.

Les radicaux R₁ et R₂ représentent de préférence CF₃.
Les radicaux Z₁ et Z₂ représentent de préférence la pipéridine ou la méthionamide.
De préférence, les radicaux R₁ et R₂ sont identiques.

Lorsqu'au moins l'un des radicaux R₁ ou R₂ est différent de l'atome d'hydrogène, les composés de formule (I) sont de plus des composés nouveaux.

L'invention a donc également pour objet des composés répondant à la formule (la) suivante : 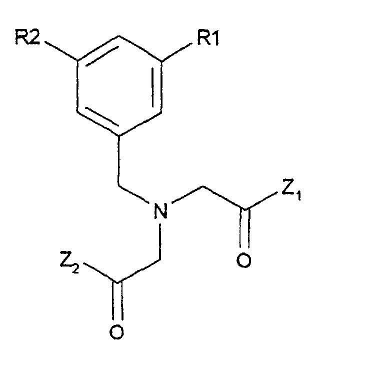 dans laquelle :
- R₁ et R₂, identiques ou différents sont choisis parmi l'hydrogène ou les radicaux CH₃, CF₃, OCH₃ et OH, sous réserve que l'un au moins des radicaux R₁ et R₂ est différent de l'hydrogène;
- Z₁ et Z₂ ont la même signification que dans la formule (I) ci-dessus.

Les composés de formule (I) peuvent être préparés selon le schéma de synthèse générale suivant :

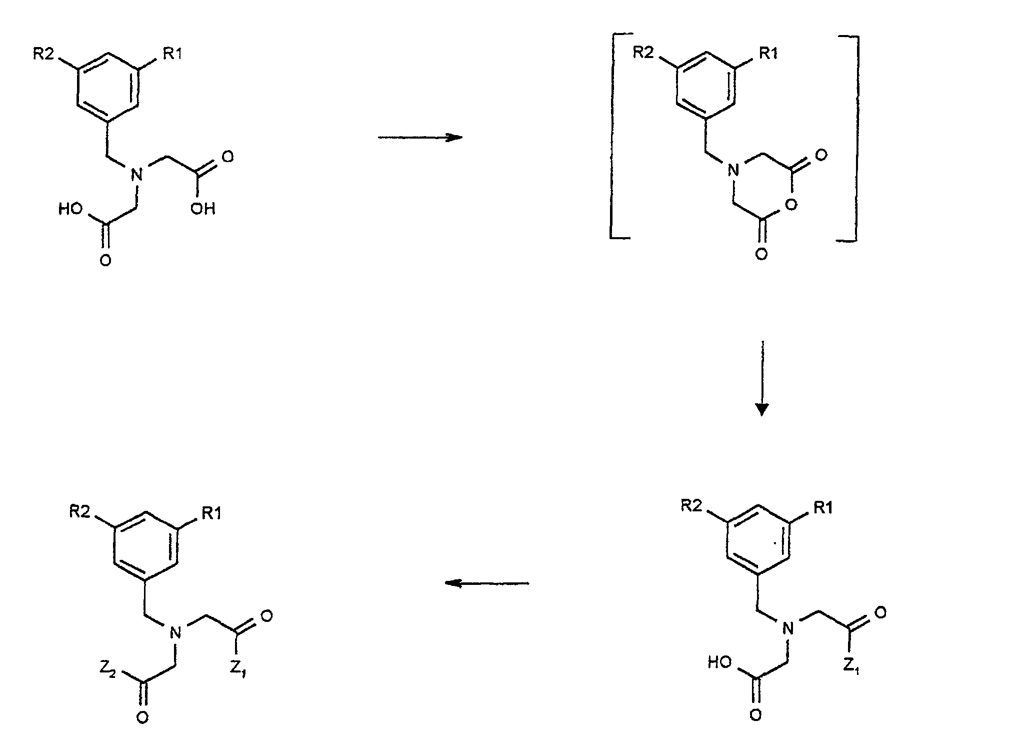

D'une manière générale, le procédé de préparation des composés de formule (I) consiste en un couplage classique entre un diacide et deux amines ou alcools.
En particulier, on peut, dans une première étape, faire réagir ledit diacide avec un agent déshydratant de manière à obtenir un anhydride intermédiaire permettant le couplage avec la première amine ou le premier alcool, puis, dans une deuxième étape, ajouter un agent de couplage, permettant le couplage dudit diacide avec la deuxième amine ou le deuxième alcool.

On donne ci-après, à titre indicatif mais non limitatif, un procédé général permettant la préparation des composés de formule (I).

Dans une première étape, on peut préparer le diacide selon le schéma suivant :

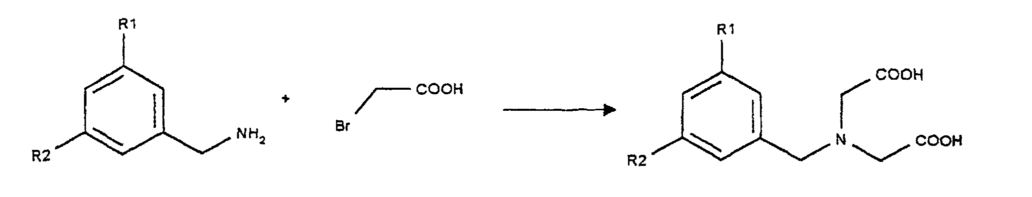

La benzylamine, éventuellement disubstituée en position 3 et/ou 5, est solubilisée dans un solvant protique comme l'éthanol. On additionne un excès d'au moins 2 équivalents de bromoacétate de sodium. Le pH du milieu est ajusté à 10 par addition d'une base forte. Le mélange est agité entre 30 et 60°C, par exemple 35-45°C, pendant 2 à 12 heures, par exemple 5-7 heures, en maintenant le pH entre 9 et 11.
Le milieu est alors refroidi et lavé par un solvant organique. La solution aqueuse restante est réacidifiée. On obtient le diacide sous forme d'un précipité que l'on filtre et sèche.

Dans une deuxième étape, on peut préparer le diamide selon le schéma suivant :

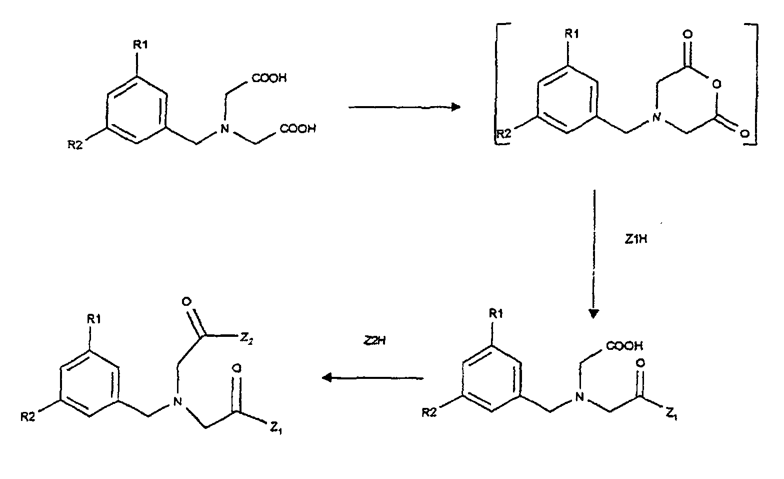

Le diacide est solubilisé dans un solvant aprotique dipolaire anhydre comme le DMF (diméthylformamide) en présence d'un agent déshydratant, par exemple l'EDCL (N-(3-diméthylaminopropyl)-N'-éthylcarbodiimide chlorhydrate) de façon à générer l'anhydride in situ. Le milieu est agité pendant 1 à 6 heures entre 20 et 40°C sous atmosphère inerte.
Un équivalent de la première amine ou alcool Z₁H est alors additionné. Le mélange est agité sous atmosphère inerte pendant 10 à 30 heures, à une température d'environ 20-40°C.
On additionne alors un équivalent de la deuxième amine ou alcool Z₂H en présence d'une quantité catalytique d'une amine non nucléophile comme la triéthylamine ou la diisopropyléthylamine, et d'un équivalent d'agent de couplage comme le PyBOP (hexafluorophosphate de (benzotriazol-1-yloxy)-tripyrrolidino-phosphonium).
Le mélange est ensuite agité sous atmosphère inerte entre 20 et 40°C pendant environ 10 à 30 heures. Le milieu réactionnel est alors acidifié et la phase aqueuse est extraite trois fois par un solvant organique.
Les différentes phases organiques sont collectées puis lavées successivement par des solutions aqueuses acides, basiques puis neutres. Après séchage, filtration et évaporation, le diamide formé est purifié par chromatographie ou cristallisation.

Parmi les amines susceptibles d'être employées, on peut citer les composés de formule suivantes, ainsi que leurs dérivés :

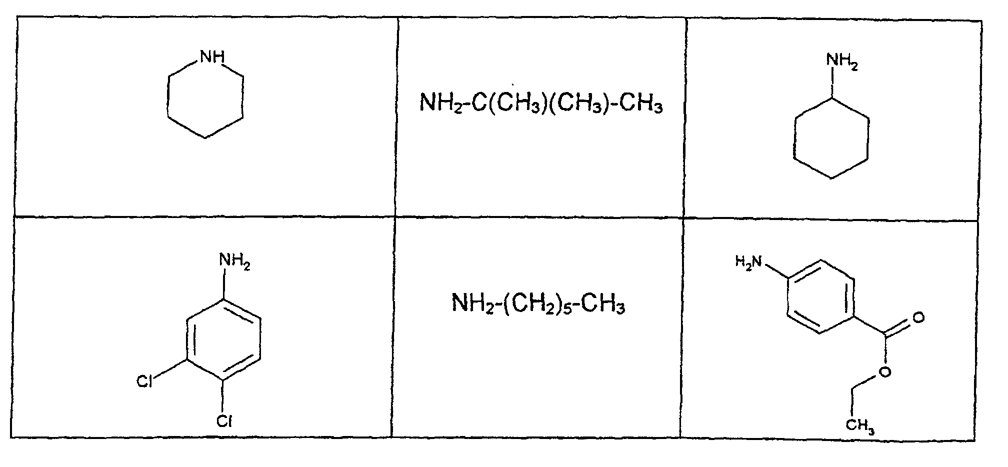

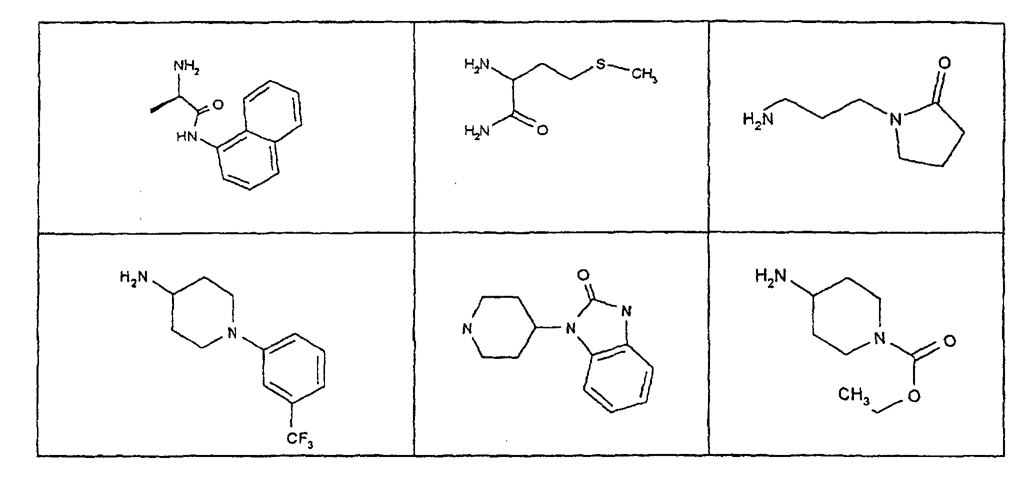

Parmi les composés de formule (I) préférés selon l'invention, on peut citer :
- le 2-[(3,5-bis-trifluoromethyl-benzyl)-hexylcarbamoylmethyl-amino]-N-(3,4-dichloro-phenyl)-acétamide;
- le 2-{2-[(3,5-bis-trifluoromethyl-benzyl)-2-oxo-2-piperidin-1-yl-éthyl-amino]-acétylamino}-4-méthylsulfanyl-butyramide;
- le 2-[(3,5-bis-trifluoromethyl-benzyl)-hexylcarbamoylmethyl-amino]-N-(tert-butyl)-acétamide.

Les composés de formule (I) peuvent notamment être employés, seul ou en mélange, dans une composition qui comprend un milieu physiologiquement acceptable, notamment dans une composition cosmétique qui comprend donc par ailleurs un milieu cosmétiquement acceptable.

En particulier, le/les composé(s) de formule (I) ainsi que les compositions les comprenant peuvent être destinés à prévenir et/ou traiter les désordres cutanés, et notamment les irritations cutanées, les dartres, les rougeurs, les sensations dysesthésiques, les sensations d'échauffement et/ou les prurits de la peau et/ou des muqueuses.
Plus particulièrement, ils peuvent être destinés à traiter les peaux sensibles.

La quantité de composé de formule (I) présent dans la composition selon l'invention est bien entendu fonction de l'effet recherché. Ainsi, la composition peut comprendre au moins un composé de formule (I) en une quantité de 0,001 à 20% du poids total de la composition, notamment en une quantité de 0,01 à 10% en poids, et préférentiellement en une quantité de 0,1 à 5% en poids du poids total de la composition.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.
Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

D'une manière générale, ce milieu peut être anhydre ou aqueux. Il peut ainsi comprendre une phase aqueuse et/ou une phase grasse.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine considéré. L'émulsionnant et le coémulsionnant peuvent être présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.
Lorsque la composition est une solution ou un gel huileux, la phase grasse peut représenter plus de 90 % du poids total de la composition.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de Camauba ou de paraffine. On peut ajouter à ces huiles des alcools gras et des acides gras (acide stéarique par exemple).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques, les conservateurs, les antioxydants, les solvants, les parfums, les charges, les pigments, les filtres, les absorbeurs d'odeur et les colorants. Ces adjuvants, selon leur nature, peuvent être introduits dans la phase grasse, dans la phase aqueuse et/ou dans des sphérules lipidiques.
Comme solvants utilisables dans l'invention, on peut citer les alcools en C1-C6, notamment l'éthanol et l'isopropanol, le propylène glycol.
Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles.
Comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut comprendre des actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides. Parmi les actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Dans le cadre de l'invention, la composition peut comprendre, en association avec le ou les composés de formule (I), des agents actifs connus destinés notamment à la prévention et/ou au traitement des affections cutanées.
Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

De plus, ainsi que précédemment mentionné, les composés de formule (I) peuvent être associés à des produits à effet irritant, notamment des actifs, utilisés couramment notamment dans le domaine cosmétique. En effet, la présence des composés de formule (I) peut permettre d'atténuer fortement, voire de supprimer, cet effet irritant. Cela permet en outre d'augmenter la quantité de produit à effet irritant par rapport à la quantité de produit normalement utilisée, en vue d'une efficacité améliorée.

Comme produits à effet irritant, on peut citer par exemple les tensioactifs (ioniques ou non ioniques), les conservateurs, les solvants organiques ou les actifs comme les α-hydroxyacides (acide citrique, malique, glycolique, tartrique, mandélique, lactique), les β-hydroxyacides (l'acide salicylique et ses dérivés), les α-cétoacides, les β-cétoacides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

Dans un autre mode particulier de mise en oeuvre de l'invention, la composition selon l'invention peut en outre comprendre au moins un composé diminuant la synthèse, la libération et/ou l'activité d'au moins un médiateur de l'inflammation. Parmi ces composés, on peut citer tous ceux énumérés dans la demande FR2746647 au nom de la demanderesse, dont le contenu est incorporé ci-après par référence. On peut notamment citer les antagonistes de substance P et/ou de CGRP, les inhibiteurs de NO-synthase, les antagonistes de bradykinine, les antagonistes de cytokines, les antagonistes d'histamine et/ou les antagonistes du facteur de nécrose tumorale de type α (TNFα). Ces composés peuvent être présents en une quantité représentant de 0,001% à 10% du poids total de la composition et préférentiellement en une quantité représentant de 0,01% à 2% du poids total de la composition.

Les compositions selon l'invention peuvent se présenter sous la forme de crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires); de fonds de teint fluides, de laits de démaquillage, de laits corporels de protection ou de soin, de laits anti-solaires; de lotions, gels ou mousses pour le soin de la peau, comme des lotions de nettoyage, des lotions anti-solaires, des lotions de bronzage artificiel; des compositions pour le bain, des compositions désodorisantes comprenant un agent bactéricide; des gels ou lotions après-rasage; des crèmes épilatoires; des compositions contre les piqûres d'insectes; des compositions anti-douleur; des compositions pour traiter certains désordres cutanés tels que l'eczéma, la rosasée, le psoriasis, les lichens, les prurits sévères.
Les compositions selon l'invention peuvent également se présenter sous forme de compositions solides constituant des savons ou des pains de nettoyage.
Les compositions peuvent aussi être conditionnées sous forme de composition pour aérosol comprenant également un agent propulseur sous pression.
Les compositions peuvent aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.
Les compositions peuvent aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

La présente invention a en outre pour objet un procédé de traitement cosmétique, caractérisé par le fait que l'on applique sur la peau, sur les cheveux, et/ou sur les muqueuses, une composition cosmétique telle que décrite ci-dessus comprenant au moins un composé de formule (I) dans un milieu cosmétiquement acceptable. Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

L'invention est illustrée plus en détail dans les exemples suivants.

### Exemple 1 : Préparation du 2-[(3,5-bis-trifluorométhyl-benzyl)-hexylcarbamoylméthyl-amino]-N-(3,4-dichloro-phényl)-acétamide

### 1ère étape: synthèse de l'acide 3,5-bis(trifluoromethyl)benzylamino N,N-diacétique

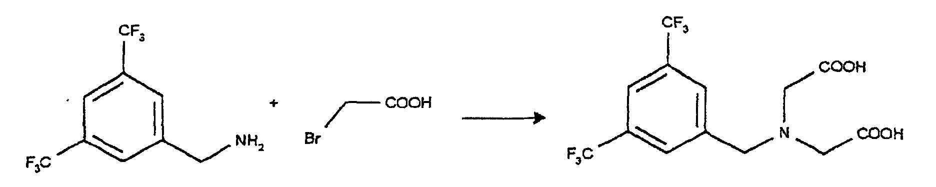

On solubilise 15 g (50 mmoles) de 3,5-bis(trifluorométhyl)benzylamine dans 40 ml d'éthanol. On additionne goutte à goutte un mélange de 13,7 g (100 mmoles) d'acide bromoacétique et 8,3 g (100 mmoles) de NaHCO₃ en solution dans 10 ml d'eau. Le pH du milieu est ajusté à 10 par addition de lessive de soude. Le mélange est agité à 40°C pendant 6 heures en maintenant le pH à 10 par addition de soude.
Le milieu est alors refroidi et lavé trois fois par 50 ml de CH₂Cl₂. La solution aqueuse restante est réacidifiée par HCI jusqu'à un pH de l'ordre de 1-2.
Le produit précipité est filtré. Le solide obtenu est séché sous vide, à 50°C, en présence de P₂O₅.
On obtient 18 g de produit se présentant sous forme de poudre blanche (rendement de 100%).

Sa caractérisation par RMN ¹H (200 MHz, DMSO de) donne le résultat suivant :
δ ppm: 3.5 (4H, s, 2H₁, 2H₂); 4.1 (2H, s, 2H₁); 8.0 (1H, s, H₇); 8.2 (2H, s, H₅,H₉) 12.5 (2H, s élargi, COOH).

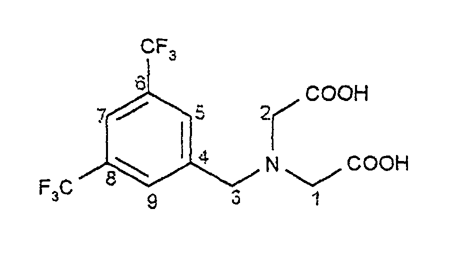

### 2ème étape: synthèse du 2-[(3,5-bis-trifluoromethyl-benzyl)-hexylcarbamoylméthyl-amino]-N-(3,4-dichloro-phenyl)-acétamide

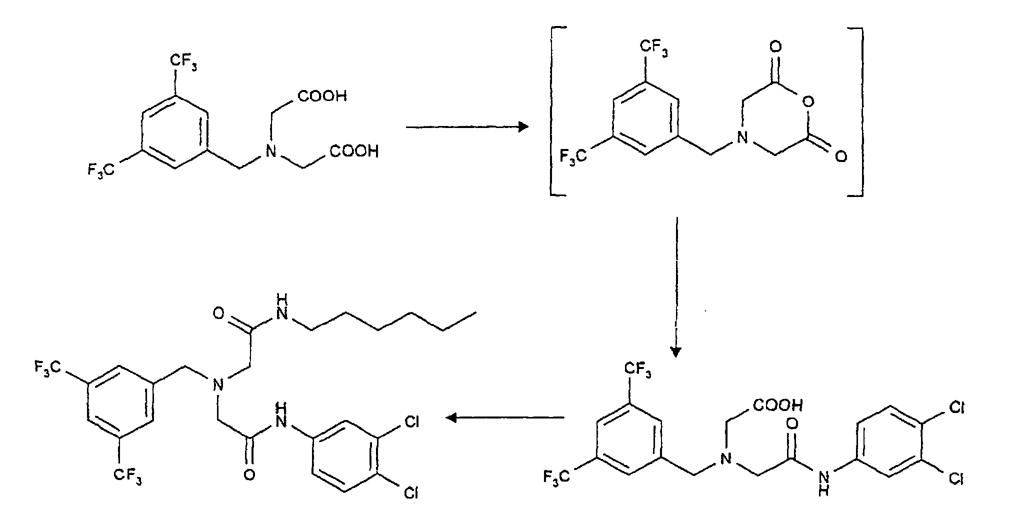

On solubilise 300 mg de diacide préparé à l'étape 1, dans 15 ml de DMF anhydre.
On ajoute 168 mg (1,05 équivalents) d'EDCI (N-(3-diméthylaminopropyl)-N'-éthylcarbodiimide chlorydrate) et on maintient l'agitation pendant 4 heures à température ambiante sous atmosphère inerte (argon).
On ajoute 135 mg (1 eq.) de 3,4-dichloroaniline en solution dans 5 ml de DMF et on agite le mélange à température ambiante sous argon pendant 20 heures.
On ajoute ensuite, à température ambiante:
- 84 mg (1 eq.) d'hexylamine en solution dans 5 ml de DMF,
- 300 µL (0.2 eq.) de diisopropyle éthylamine, et
- 435 mg de PyBOP (1 eq.) (hexafluorophosphate de (benzotriazol-1-yloxy)-tripyrrolidinophosphonium) en solution dans 5 ml de DMF anhydre.
Le mélange est ensuite agité à température ambiante sous argon pendant 20 heures.

On acidifie le mélange réactionnel par ajout de HCI 10% et la phase aqueuse est extraite trois fois par 20 ml d'acétate d'éthyle. Les différentes phases organiques sont collectées puis lavées successivement par des solutions d'HCl 10%, NaHCO₃ saturée et enfin NaCI saturée. Après séchage sur Na₂SO₄, filtration et évaporation, on récupère 450 mg d'une huile jaune. Cette huile est purifiée par chromatographie "flash" sur gel de silice (CH₂Cl₂/MeOH=1%).

Après purification, on obtient 304 mg de produit ayant l'aspect d'un solide jaune, soit 62% de rendement.

Sa caractérisation par RMN ¹H (200 MHz) dans CDCl₃ donne un spectre conforme à la structure du produit attendu.
δ ppm: 0.78 (3H, t, 3H₁₂), 1.1 (6H, m, 2H₁₁, 2H₁₀, 2H₉), 1.4 (2H, m, 2H₈), 3.15 (2H, q, 2H₇), 3.3 (2H, s, 2H₄), 3.35 (2H, s, 2H₃), 3.9 (2H, s, 2H₁₉) 5.8 (1H, t, H6) 7.3 (1H, s, H_{aryl}), 7.37 (1H, d, H_{aryl}) 7.73 (1H, s, H_{aryl}), 7.77 (2H, s, H_{aryl}), 7.88 (1H, d, H_{aryl})

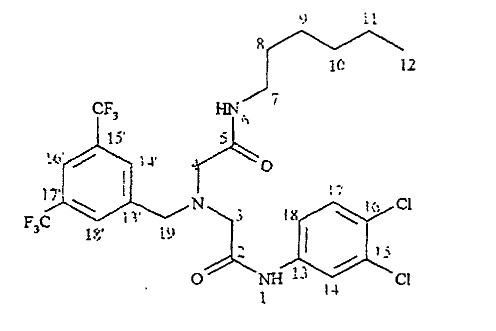

### Exemple 2

Selon le même procédé qu'à l'exemple 1, on prépare deux autres composés à partir des composés de départ donnés ci-dessous.

La structure des composés obtenus a été vérifiée par couplage HPLC / spectrométrie de masse en utilisant la technique d'ionisation par electrospray à pression atmosphérique.
Pour ces deux composés, on indique ci-dessous, la masse observée en ESI-MS.

### 1/Composés de départ :

- 3,5-bis(trifluorométhyl)benzylamine
- composé de formule : 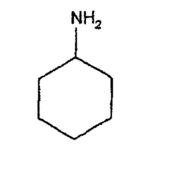
- composé de formule :

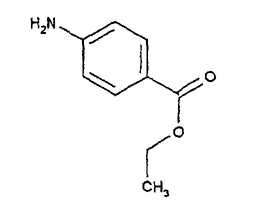

On obtient alors le composé de formule :

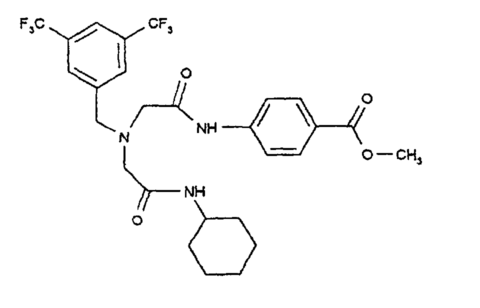

Sa masse observée est de SM (ES+/-): 574,4 g (M+ Na)

### 2/Composés de départ :

- 3,5-bis(trifluorométhyl)benzylamine
- composé de formule : 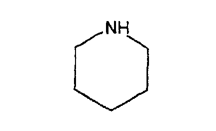
- composé de formule : NH₂-C(CH₃)(CH₃)-CH₃

On obtient alors le composé final de formule :

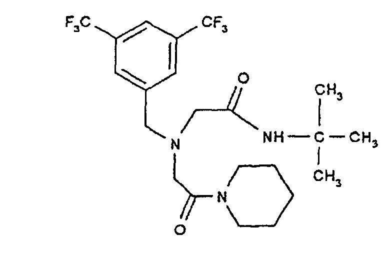

Sa masse observée est de SM (ES+/-): 504,4 g (M+Na)

### Exemple 3

Selon le même procédé, on prépare également les composés répondant aux formules suivantes :

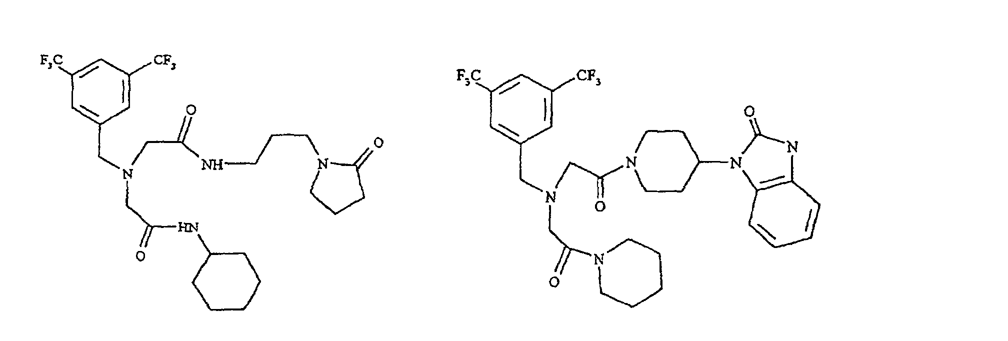

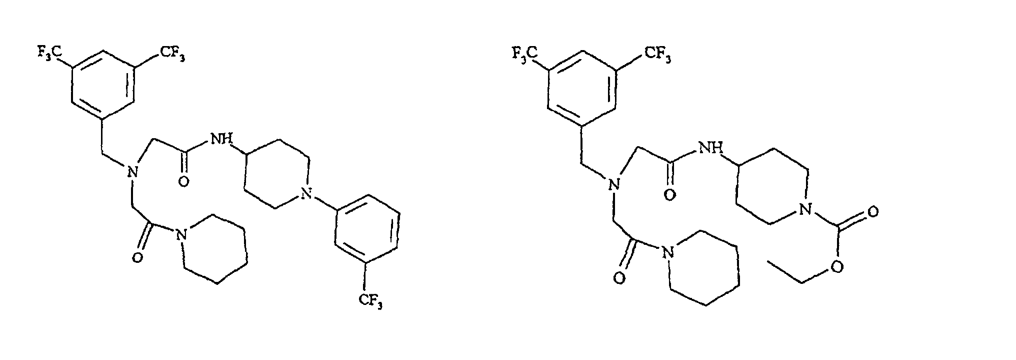

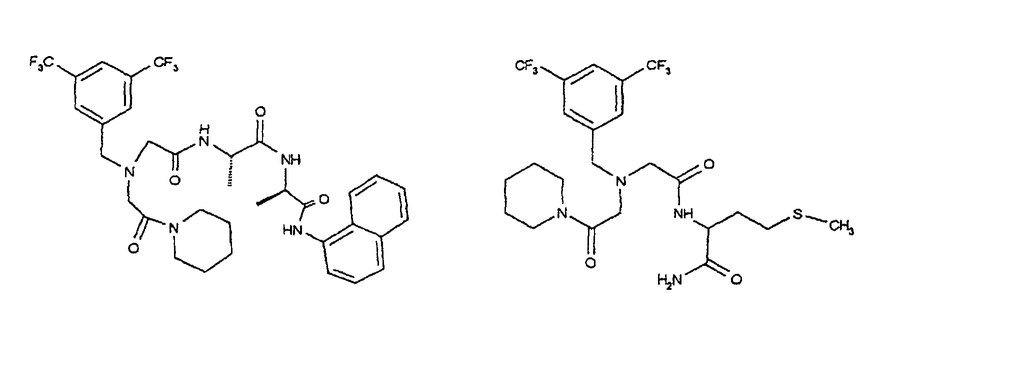

### Exemple 4

On prépare une crème de soin du visage (émulsion huile dans eau) comprenant :

| | |
|---|---|
| Composé de l'exemple 1 | 1,00 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | qs |
| Parfum | qs |
| Conservateur | qs |
| Eau | qsp 100 % |

### Exemple 5

On prépare un shampooing comprenant :

| | |
|---|---|
| Composé de l'exemple 1 | 0,50 |
| Hydroxypropylcellulose (Klucel H de la société Hercules) | 1,00 |
| Parfum | qs |
| Conservateur | qs |
| Eau | qsp 100 % |

### Exemple 6

On prépare un gel apaisant la douleur comprenant :

| | |
|---|---|
| Composé de l'exemple 1 | 1,00 |
| Hydroxypropylcellulose (Klucel H de la société Hercules) | 1,00 |
| Antioxydant | 0,05 |
| Chlorhydrate de lidocaïne | 2,00 |
| Isopropanol | 40,00 |
| Conservateur | qs |
| Eau | qsp 100 % |

### Exemple 7

On prépare une crème de soin après soleil (émulsion huile dans eau) comprenant:

| | |
|---|---|
| Composé de l'exemple 1 | 0,80 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | qs |
| Parfum | qs |
| Conservateur | qs |
| Eau | qsp 100 % |

### Exemple 8

On prépare un gel de soin du visage comprenant :

| | |
|---|---|
| Composé de l'exemple 1 | 0,50 |
| Eau thermale de Vichy | 10,00 |
| Hydroxypropylcellulose (Klucel H vendu par la société Hercules) | 1,00 |
| Antioxydant | qs |
| Isopropanol | 40,00 |
| Conservateur | qs |
| Eau | qsp 100 % |

### Exemple 9

On prépare une crème de soin après-soleil (émulsion huile dans eau) comprenant

| | |
|---|---|
| Composé de l'exemple 1 | 5,00 |
| Eau thermale de Vichy | 10,00 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | qs |
| Parfum | qs |
| Conservateur | qs |
| Eau | qsp 100 % |

### Exemple 10

On prépare un gel apaisant la douleur comprenant :

| | |
|---|---|
| Composé de l'exemple 1 | 5,00 |
| Spantide II | 0,05 |
| Hydroxypropylcellulose (Klucel H vendu par la société Hercules) | 1,00 |
| Antioxydant | qs |
| Chlorhydrate de lidocaïne | 2,00 |
| Isopropanol | 40,00 |
| Conservateur | qs |
| Eau | qsp 100 % |

## Revendications

1. Composition comprenant, dans un milieu physiologiquement acceptable, au moins un composé répondant à la formule (I) suivante : 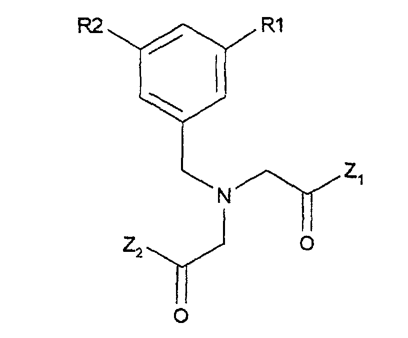 dans laquelle :
- R₁ et R₂, identiques ou différents sont choisis parmi l'hydrogène ou les radicaux CH₃, CF₃, OCH₃ et OH,
- Z₁ est choisi parmi les radicaux NX₁X₂ et OX₁, et Z₂ est choisi parmi les radicaux NX₃X₄ et OX₃, dans lesquels :
- X₁ et X₃, identiques ou différents, représentent :
- un radical alkyle en C₁-C₁₂, éventuellement substitué par un ou plusieurs groupements OH, NH₂, SH, CN, CF₃, halogène, COOH, CONHR', COOR', OR', SR' avec R' représentant un alkyle en C₁-C₄; ou éventuellement substitué par un ou plusieurs cycles en C₃-C₇ aliphatiques ou aromatiques, éventuellement hétérocycliques; ou
- un radical aryle, éventuellement substitué par un ou plusieurs groupements OH, NH₂, SH, CN, CF₃, halogène, COOH, CONHR', COOR', OR', SR' avec R' représentant un alkyle en C₁-C₄; ou éventuellement substitué par un ou plusieurs cycles en C₃-C₇ aliphatiques ou aromatiques, éventuellement hétérocycliques; et
- X₂ et X₄, identiques ou différents, représentent un atome d'hydrogène ou forment un cycle à 5 ou 6 chaînons avec X₁ et X₃ respectivement.

2. Composition selon la revendication 1, dans laquelle :
- les radicaux R₁ et R₂ représentent CF₃ et/ou
- les radicaux Z₁ et Z₂ représentent la pipéridine ou la méthionamide.

3. Composition selon l'une des revendications précédentes, dans laquelle le composé de formule (I) est choisi parmi, seul ou en mélange, les composés répondant à l'une des formules suivantes :

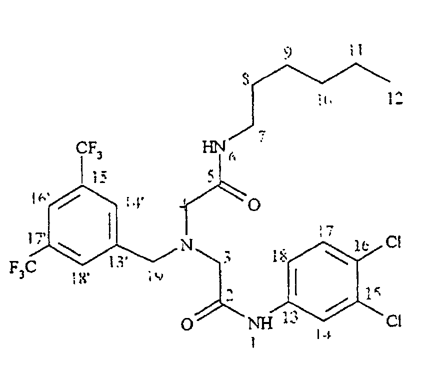

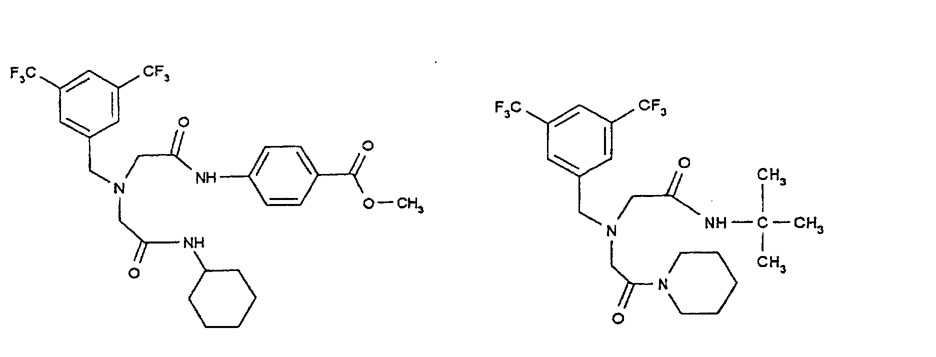

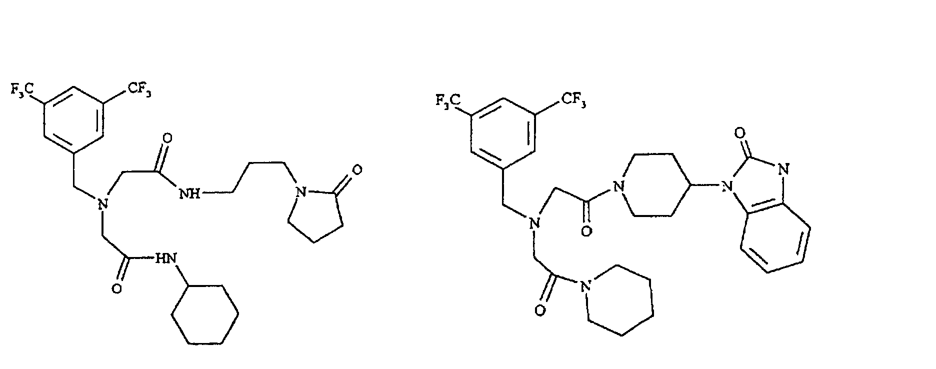

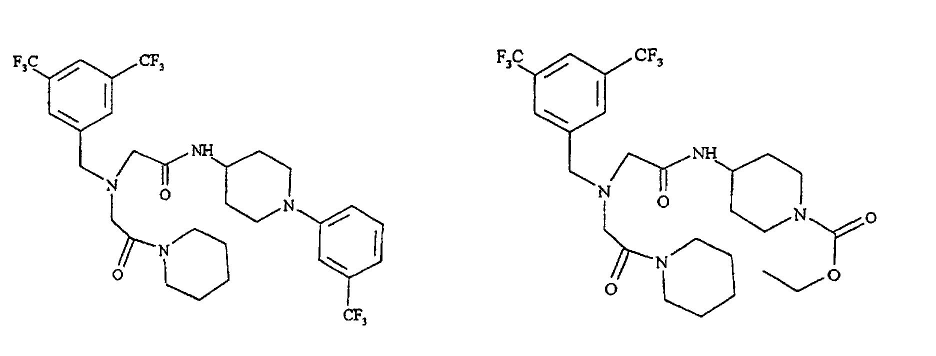

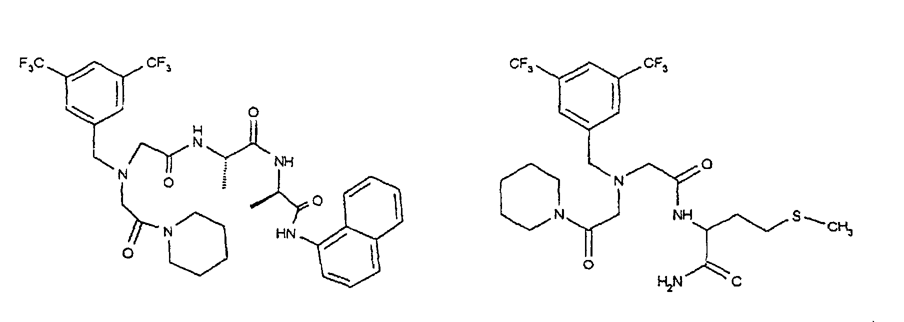

4. Composition selon l'une des revendications précédentes, dans laquelle le composé de formule (I) est présent en une quantité de 0,001 à 20% du poids total de la composition, notamment en une quantité de 0,01 à 10% en poids, et préférentiellement en une quantité de 0,1 à 5% en poids du poids total de la composition.

5. Composition selon l'une des revendications précédentes, se présentant sous forme de solution aqueuse, alcoolique, hydroalcoolique ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique; sous forme de crèmes, de gels, de mousses; sous forme de compositions pour aérosol comprenant également un agent propulseur sous pression.

6. Composition selon l'une des revendications précédentes, comprenant en outre au moins un agent actif destiné à la prévention et/ou au traitement des affections cutanées, tel que les agents modulant la différenciation et/ou la prolifération et/ou la pigmentation cutanée; les antibactériens ; les antiparasitaires; les antifongiques; les agents antiviraux ; les agents anti-inflammatoires stéroïdiens; les agents anesthésiques ; les agents antiprurigineux ; les agents kératolytiques ; les agents anti-radicaux libres; les antiséborrhéiques ; les antipelliculaires ; les antiacnéiques.

7. Composition selon l'une des revendications précédentes, comprenant en outre au moins un produit à effet irritant, tel que les tensioactifs (ioniques ou non ioniques), les conservateurs, les solvants organiques ou les actifs comme les α-hydroxyacides (acide citrique, malique, glycolique, tartrique, mandélique, lactique), les β-hydroxyacides (l'acide salicylique et ses dérivés), les α-cétoacides, les β-cétoacides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

8. Composition selon l'une des revendications précédentes, comprenant en outre au moins un composé diminuant la synthèse, la libération et/ou l'activité d'au moins un médiateur de l'inflammation.

9. Composition selon l'une des revendications précédentes, se présentant sous la forme de crèmes de nettoyage, de protection, de traitement ou de soin pour le visage, pour les mains, pour les pieds, pour les grands plis anatomiques ou pour le corps; de fonds de teint fluides, de laits de démaquillage, de laits corporels de protection ou de soin, de laits anti-solaires; de lotions, gels ou mousses pour le soin de la peau, comme des lotions de nettoyage, des lotions anti-solaires, des lotions de bronzage artificiel; des compositions pour le bain, des compositions désodorisantes comprenant un agent bactéricide; des gels ou lotions après-rasage; des crèmes épilatoires; des compositions contre les piqûres d'insectes; des compositions anti-douleur; des compositions pour traiter certains désordres cutanés; de compositions solides constituant des savons ou des pains de nettoyage; de composition pour aérosol comprenant également un agent propulseur sous pression; de composition pour soins capillaires, et notamment un shampooing, une lotion de mise en plis, une lotion traitante, une crème ou un gel coiffant, une composition de teintures éventuellement sous forme de shampooings colorants, des lotions restructurantes pour les cheveux, une composition de permanente ; une lotion ou un gel antichute, un shampooing antiparasitaire; de composition à usage bucco-dentaire, par exemple une pâte dentifrice.

10. Composition selon l'une des revendications précédentes, dans laquelle le milieu est un milieu cosmétiquement acceptable.

11. Composition selon l'une des revendications précédentes, destinée à prévenir et/ou traiter les désordres cutanés, et notamment les irritations cutanées, les dartres, les rougeurs, les sensations dysesthésiques, les sensations d'échauffement et/ou les prurits de la peau et/ou des muqueuses.

12. Composition selon l'une des revendications précédentes, destinée à traiter les peaux sensibles.

13. Composé répondant à la formule (la) suivante : 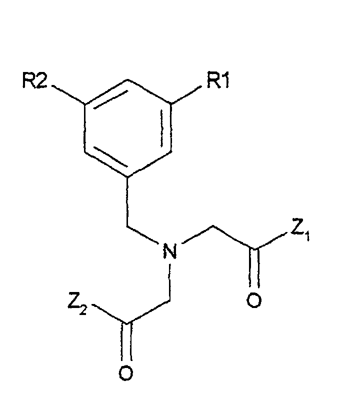 dans laquelle :
- R₁ et R₂, identiques ou différents sont choisis parmi l'hydrogène ou les radicaux CH₃, CF₃, OCH₃ et OH, sous réserve que l'un au moins des radicaux R₁ et R₂ est différent de l'hydrogène;
- Z₁ et Z₂ ont la même signification que dans la revendication 1 ci-dessus.

14. Composé selon la revendication 13, dans lequel :
- les radicaux R₁ et R₂ représentent CF₃ et/ou
- les radicaux Z₁ et Z₂ représentent la pipéridine ou la méthionamide.

15. Composé selon l'une des revendications 13 à 14, choisi parmi les composés répondant à l'une des formules suivantes :

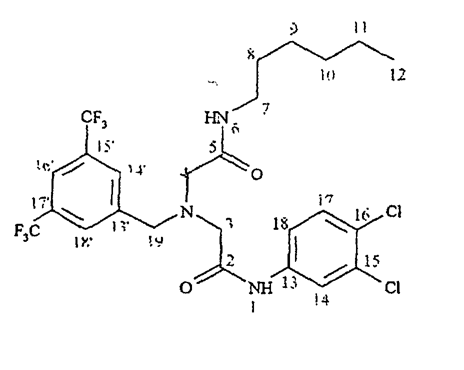

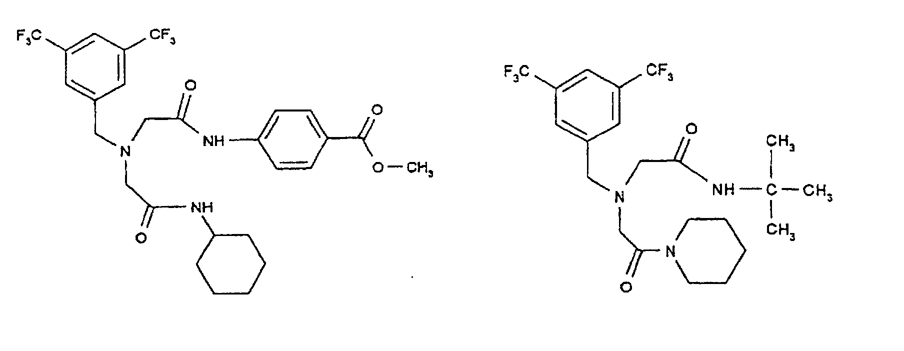

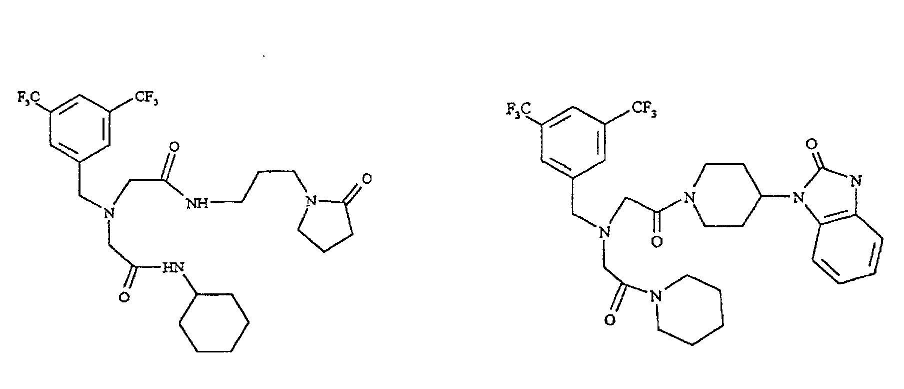

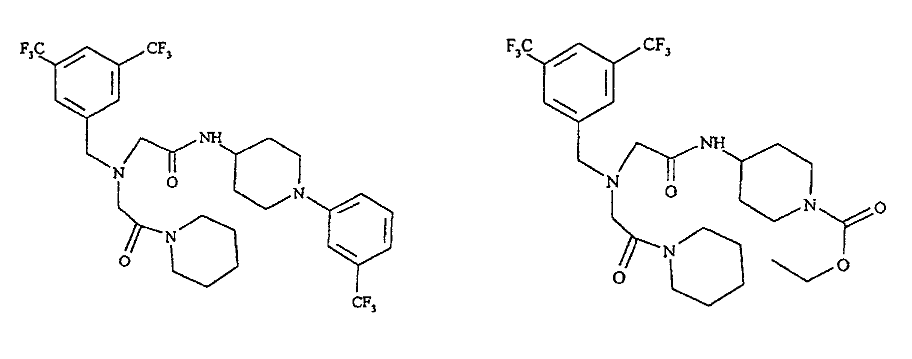

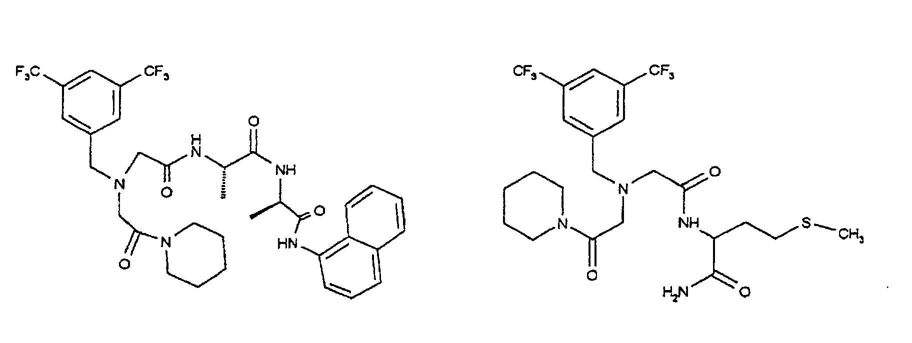

16. Procédé de préparation d'un composé de formule (Ia) selon la revendication 13, dans lequel on fait réagir un diacide avec un agent déshydratant de manière à obtenir un anhydride intermédiaire permettant le couplage avec une première amine ou un premier alcool, puis, dans une deuxième étape, on ajoute un agent de couplage, permettant le couplage dudit diacide avec une deuxième amine ou un deuxième alcool.

17. Utilisation d'au moins un composé de formule (I) telle que définie dans l'une des revendications 1 à 3, dans une composition cosmétique comprenant par ailleurs un produit à effet irritant, pour atténuer, voire supprimer ledit effet irritant.

18. Utilisation d'au moins un composé de formule (I) telle que définie dans l'une des revendications 1 à 3, dans une composition cosmétique ou pour la préparation d'une composition physiologiquement acceptable, pour prévenir et/ou traiter les désordres cutanés, et notamment les irritations cutanées, les dartres, les rougeurs, les sensations dysesthésiques, les sensations d'échauffement et/ou les prurits de la peau et/ou des muqueuses.

19. Utilisation selon la revendication 18, pour traiter les peaux sensibles.

20. Procédé de traitement cosmétique, **caractérisé par le fait que** l'on applique sur la peau, sur les cheveux et/ou sur les muqueuses, une composition cosmétique telle que définie dans l'une quelconque des revendications 1 à 12.

## Patentansprüche

1. Zusammensetzung, die in einem physiologisch akzeptablen Medium mindestens eine Verbindung der folgenden Formel (I) enthält: 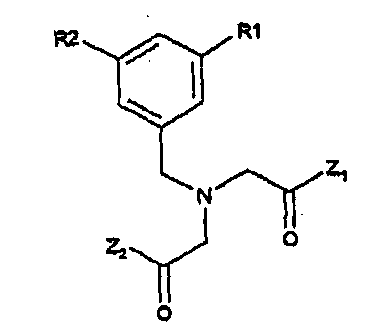 worin:
- die Gruppen R₁ und R₂, die gleich oder verschieden sind, unter Wasserstoff oder den Gruppen CH₃, CF₃, OCH₃ und OH ausgewählt sind, und
- die Gruppe Z₁ unter den Gruppen NX₁X₂ und OX₁ und die Gruppe Z₂ unter den Gruppen NX₃X₄ und OX₃ ausgewählt ist, worin bedeuten:
- X₁ und X₃, die identisch oder voneinander verschieden sind:
- eine C₁₋₁₂-Alkylgruppe, die gegebenenfalls mit einer oder mehreren Gruppen OH, NH₂, SH, CN, CF₃, Halogen, COOH, CONHR', COOR', OR' oder SR', wobei R' eine C₁₋₄-Alkylgruppe bedeutet, oder gegebenenfalls mit einer oder mehreren aliphatischen oder aromatischen cyclischen C₃₋₇-Gruppen, die gegebenenfalls heterocyclisch vorliegen, substituiert ist,
- eine Arylgruppe, die gegebenenfalls mit einer oder mehreren Gruppen OH, NH₂, SH, CN, CF₃, Halogen, COOH, CONHR', COOR', OR' oder SR', wobei R' eine C₁₋₄-Alkylgruppe bedeutet, oder gegebenenfalls mit einer oder mehreren aliphatischen oder aromatischen cyclischen C₃₋₇-Gruppen, die gegebenenfalls heterocyclisch vorliegen, substituiert ist, und
- X₂ und X₄, die identisch oder voneinander verschieden sind, Wasserstoff oder sie bilden mit den Gruppen X₁ bzw. X₃ einen 5- oder 6-gliedrigen Ring.

2. Zusammensetzung nach Anspruch 1, wobei:
- die Gruppen R₁ und R₂ CF₃ bedeuten und/oder
- die Gruppen Z₁ und Z₂ Piperidin oder Methionamid bedeuten.

3. Zusammensetzung nach einem der vorhergehenden Ansprüche, wobei die Verbindung der Formel (I) unter den Verbindungen der folgenden Formeln oder deren Gemischen ausgewählt ist:

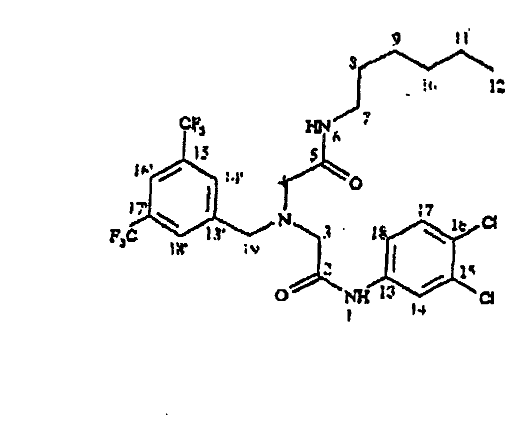

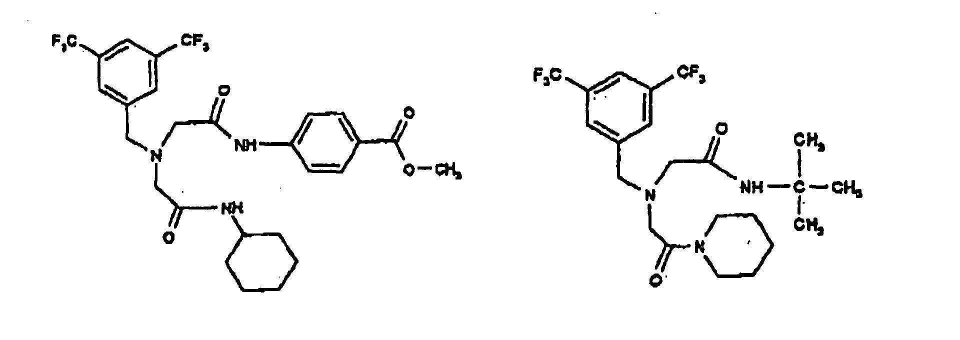

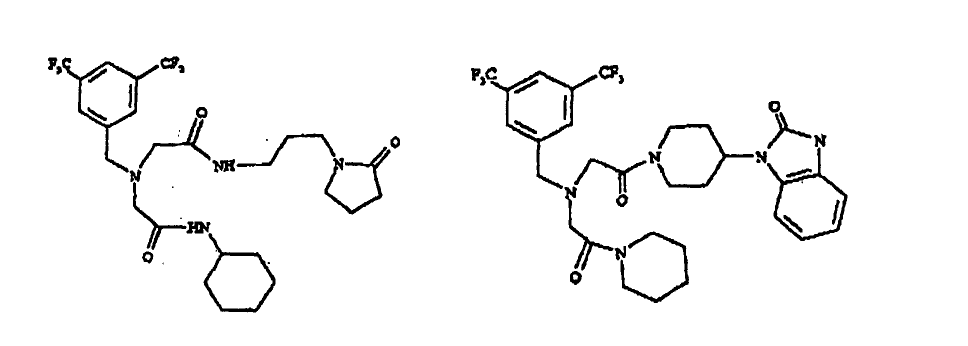

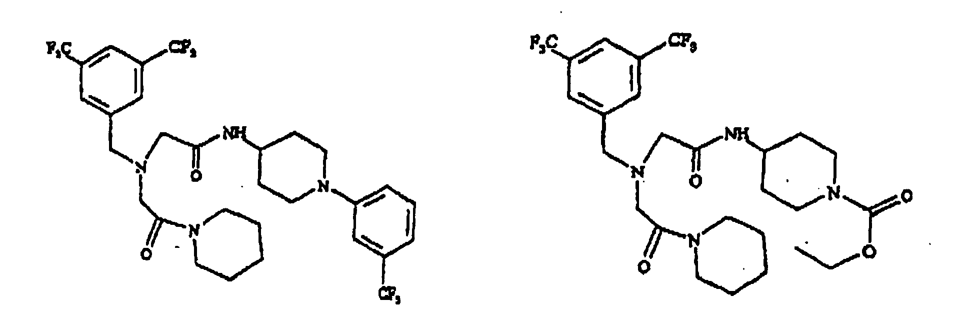

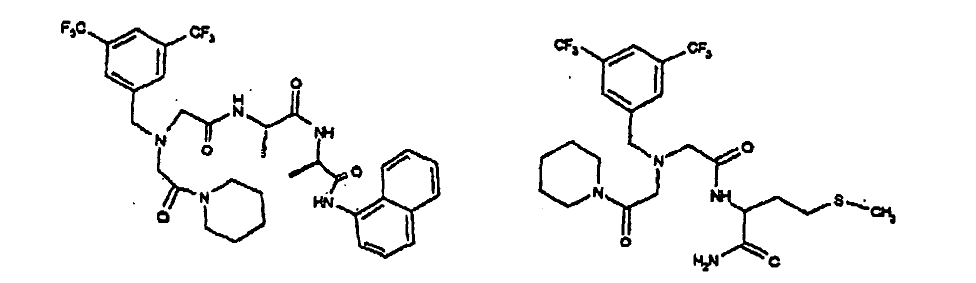

4. Zusammensetzung nach einem der vorhergehenden Ansprüche, wobei die Verbindung der Formel (I) in einer Menge von 0,001 bis 20 % des Gesamtgewichts der Zusammensetzung, und insbesondere in einer Menge von 0,01 bis 10 Gew.-% und vorzugsweise 0,1 bis 5 Gew.-% des Gesamtgewichts der Zusammensetzung vorliegt.

5. Zusammensetzung nach einem der vorhergehenden Ansprüche, die in Form von wäßrigen, alkoholischen, wäßrig-alkoholischen oder öligen Lösungen, Dispersionen vom Typ Lotion oder Serum, Emulsionen von flüssiger oder halbflüssiger Konsistenz vom Typ Milch, die durch Dispersion einer Fettphase in einer wäßrigen Phase (O/W) oder umgekehrt (W/O) hergestellt werden, Suspensionen oder Emulsionen weicher Konsistenz vom Typ einer Creme oder eines wäßrigen oder wasserfreien Gels, Mikrokapseln oder Mikropartikeln, Vesikeldispersionen vom ionischen und/oder nichtionischen Typ, Cremes, Gelen, Schäumen oder Zusammensetzungen für Aerosole, die auch ein Treibmittel unter Druck enthalten, vorliegt.

6. Zusammensetzung nach einem der vorhergehenden Ansprüche, die ferner mindestens einen Wirkstoff enthält, der zur Vorbeugung und/oder Behandlung von Hautstörungen vorgesehen ist, beispielsweise Wirkstoffe, die die Differenzierung und/oder Proliferation und/oder Pigmentierung der Haut verändern, antibakterielle Wirkstoffe, antiparasitäre Wirkstoffe, Antimykotika, antivirale Wirkstoffe, steroidale entzündungshemmende Wirkstoffe, anästhesierende Wirkstoffe, juckreizstillende Mittel, Keratolytika, Radikalfänger für freie Radikale, Antiseborrhöika, Antischuppenmittel und Wirkstoffe gegen Akne.

7. Zusammensetzung nach einem der vorhergehenden Ansprüche, die ferner mindestens ein Produkt mit reizender Wirkung enthält, wie grenzflächenaktive Stoffe (ionische oder nichtionische grenzflächenaktive Stoffe), Konservierungsmittel, organische Lösungsmittel oder Wirkstoffe, wie die α-Hydroxysäuren (Citronensäure, Äpfelsäure, Glykolsäure, Weinsäure, Mandelsäure, Milchsäure), β-Hydroxysäuren (Salicylsäure und ihre Derivate), α-Ketosäuren, β-Ketosäuren, Retinoide (Retinol, Retinal, Retinsäure), Anthraline (Dioxyanthranol), Anthranoide, Peroxide (insbesondere Benzoylperoxid), Minoxidil, Lithiumsalze, Antimetaboliten, Vitamin D und seine Derivate, Haarfärbemittel oder Farbstoffe für das Haar (p-Phenylendiamin und seine Derivate, Aminophenole), parfümierende alkoholische Lösungen (Parfums, Eau de Toilette, After Shave, Deodorants), Antitranspirantien (verschiedene Aluminiumsalze), Wirkstoffe zur Haarentfernung oder für Dauerwellen (Thiole) und depigmentierende Wirkstoffe (Hydrochinon).

8. Zusammensetzung nach einem der vorhergehenden Ansprüche, die mindestens eine Verbindung enthält, die die Synthese, Freisetzung und/oder Aktivität mindestens eines Entzündungsmediators vermindert.

9. Zusammensetzung nach einem der vorhergehenden Ansprüche, die als Creme zur Reinigung, zum Schutz, zur Behandlung oder zur Pflege des Gesichts, der Hände, der Füße, der Körperfalten oder des Körpers, flüssiges Make-up, Milch zum Abschminken, Körpermilch zum Schutz oder zur Pflege, After-sun-Produkt in Form von Milch, Lotion, Gel oder Schaum zur Pflege der Haut, wie Reinigungslotionen, Sonnenschutzlotionen oder Lotionen zur künstlichen Bräunung, Zusammensetzung für das Bad, desodorierende Zusammensetzung, die ein Bakterizid enthält, nach der Rasur anzuwendendes Gel oder nach der Rasur anzuwendende Lotion, Haarentfernungscreme, Zusammensetzung gegen Insektensuche, schmerzstillende Zusammensetzung und Zusammensetzung zur Behandlung verschiedener Hautstörungen, feste Zusammensetzung, die als Seife oder Stückseife vorliegt, Zusammensetzung für Aerosole, die auch ein Treibmittel unter Druck enthält, Zusammensetzung zur Pflege der Haare, insbesondere als Haarwaschmittel, Lotion für Wasserwellen, Behandlungslotion, Frisiercreme, Frisiergel, Zusammensetzung zum Färben gegebenenfalls in Form von färbenden Haarwaschmittel, restrukturierende Lotion für das Haar und Zusammensetzung für permanente Verformung, Lotion oder Gel gegen Haarausfall, antiparasitäres Haarwaschmittel oder Zusammensetzung zur Mund- und Zahnpflege, beispielsweise als Zahncreme, vorliegt.

10. Zusammensetzung nach einem der vorhergehenden Ansprüche, wobei das Medium ein kosmetisch akzeptables Medium ist.

11. Zusammensetzung nach einem der vorhergehenden Ansprüche, die dazu vorgesehen ist, Hautstörungen und insbesondere Hautreizungen, Flechten, Rötungen, dysesthesische Empfindungen, Hitzegefühle und/oder Juckreiz der Haut und/oder der Schleimhäute vorzubeugen und/oder zu behandeln.

12. Zusammensetzung nach einem der vorhergehenden Ansprüche zur Behandlung von empfindlicher Haut.

13. Verbindung der folgenden Formel (Ia): 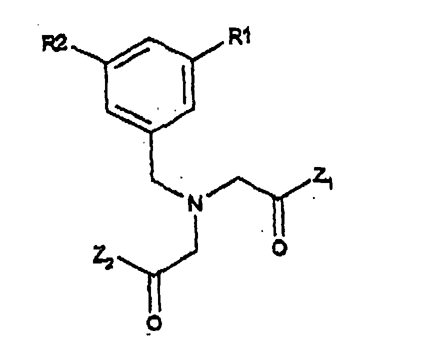 worin:
- die Gruppen R₁ und R₂, die gleich oder verschieden sind, unter Wasserstoff oder den Gruppen CH₃, CF₃, OCH₃ und OH ausgewählt sind, mit der Maßgabe, daß mindestens eine der Gruppen R₁ und R₂ von Wasserstoff verschieden ist, und
- die Gruppen Z₁ und Z₂ die oben in Anspruch 1 angegebenen Bedeutungen aufweisen.

14. Verbindung nach Anspruch 13, wobei
- die Gruppen R₁ und R₂ CF₃ bedeuten und/oder
- die Gruppen Z₁ und Z₂ Piperidin oder Methionamid bedeuten.

15. Verbindung nach einem der Ansprüche 13 bis 14, die unter den Verbindungen der folgenden Formeln ausgewählt ist:

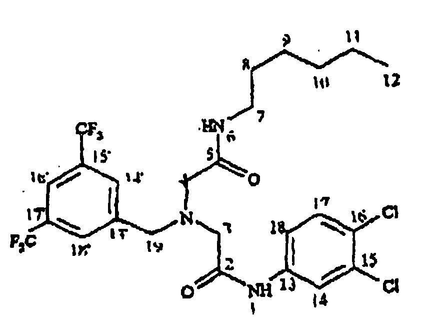

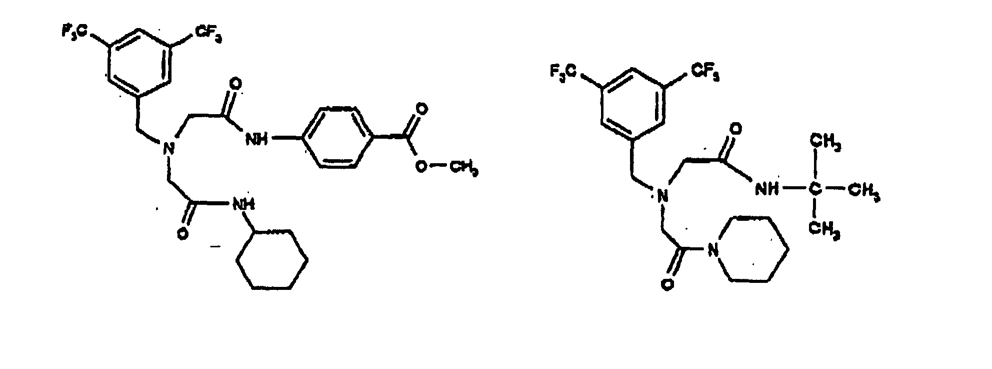

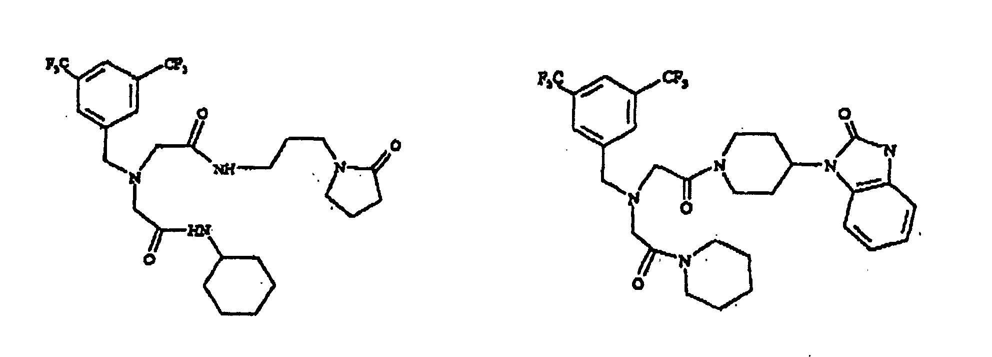

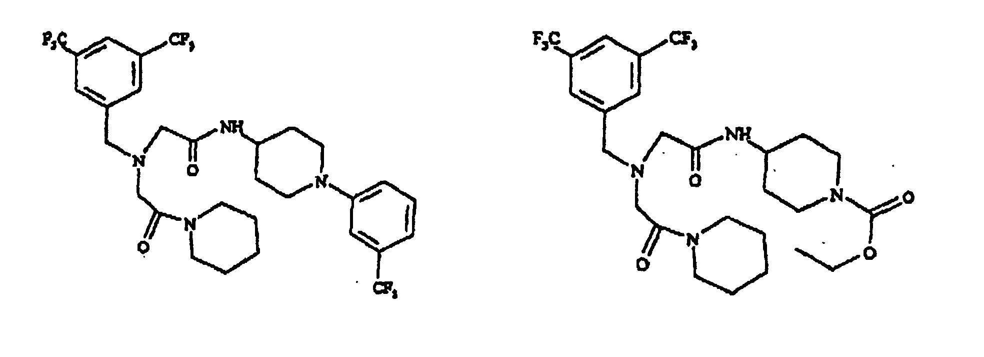

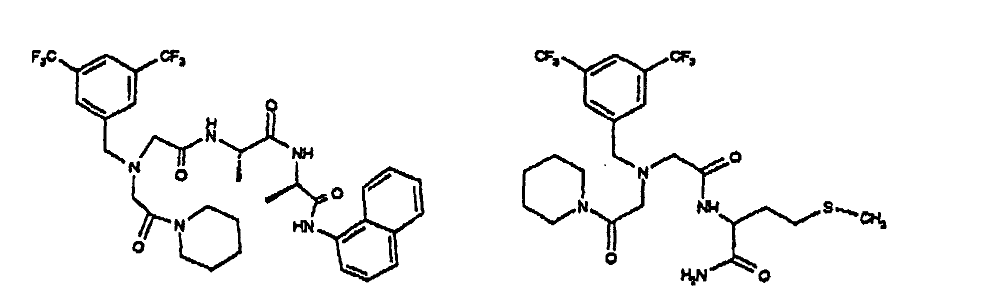

16. Verfahren zur Herstellung einer Verbindung der Formel (Ia) nach Anspruch 13, wobei eine Disäure mit einem Dehydratisierungsmittel so umgesetzt wird, daß als Zwischenprodukt ein Anhydrid gebildet wird, das die Kupplung mit einem ersten Amin oder einem ersten Alkohol ermöglicht, und dann in einem zweiten Schritt ein Kupplungsmittel zugegeben wird, das die Kupplung der Disäure mit einem zweiten Amin oder einem zweiten Alkohol ermöglicht.

17. Verwendung mindestens einer Verbindung der Formel (I) nach einem der Ansprüche 1 bis 3 in einer kosmetischen Zusammensetzung, die auch ein Produkt mit reizender Wirkung enthält, um die reizende Wirkung abzuschwächen oder zu beseitigen.

18. Verwendung mindestens einer Verbindung der Formel (I) nach einem der Ansprüche 1 bis 3 in einer kosmetischen Zusammensetzung oder zur Herstellung einer physiologisch akzeptablen Zusammensetzung, um Störungen der Haut vorzubeugen und/oder sie zu behandeln, insbesondere Hautreizungen, Flechten, Rötungen, dysesthesische Empfindungen, Hitzegefühle und/oder Juckreiz der Haut und/oder der Schleimhäute.

19. Verwendung nach Anspruch 18 zur Behandlung empfindlicher Haut.

20. Verfahren zur kosmetischen Behandlung, **dadurch gekennzeichnet, daß** auf die Haut, die Haare und/oder die Schleimhäute eine kosmetische Zusammensetzung nach einem der Ansprüche 1 bis 12 aufgebracht wird.

## Claims

1. Composition comprising, in a physiologically acceptable medium, at least one compound corresponding to the following formula (I): 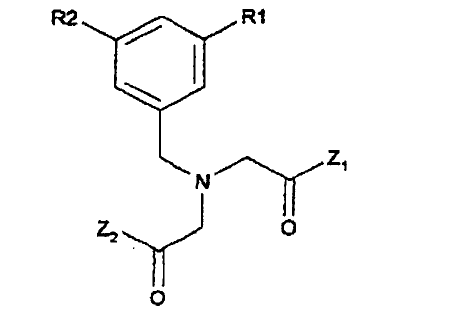 in which:
- R₁ and R₂, which are identical or different, are chosen from hydrogen or CH₃, CF₃, OCH₃ and OH radicals,
- Z₁ is chosen from NX₁X₂ and OX₁ radicals and Z₂ is chosen from NX₃X₄ and OX₃ radicals, in which:
- X₁ and X₃, which are identical or different, represent:
- a C₁-C₁₂ alkyl radical which is optionally substituted by one or more OH, NH₂, SH, CN, CF₃, halogen, COON, CONHR', COOR', OR' or SR' groups with R' representing a C₁-C₄ alkyl or which is optionally substituted by one or more aliphatic or aromatic, optionally heterocyclic, C₃-C₇ rings; or
- an aryl radical which is optionally substituted by one or more OH, NH₂, SH, CN, CF₃, halogen, COOH, CONHR', COOR', OR' or SR' groups with R' representing a C₁-C₄ alkyl or which is optionally substituted by one or more aliphatic or aromatic, optionally heterocyclic, C₃-C₇ rings; and
- X₂ and X₄, which are identical or different, represent a hydrogen atom or form a 5- or 6-membered ring with X₁ and X₃ respectively.

2. Composition according to Claim 1, in which:
- the R₁ and R₂ radicals represent CF₃ and/or
- the Z₁ and Z₂ radicals represent piperidine or methionamide.

3. Composition according to either of the preceding claims, in which the compound of formula (I) is chosen, alone or as a mixture, from the compounds corresponding to one of the following formulae:

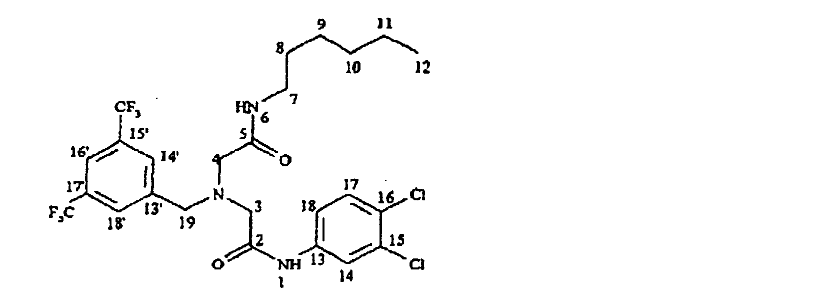

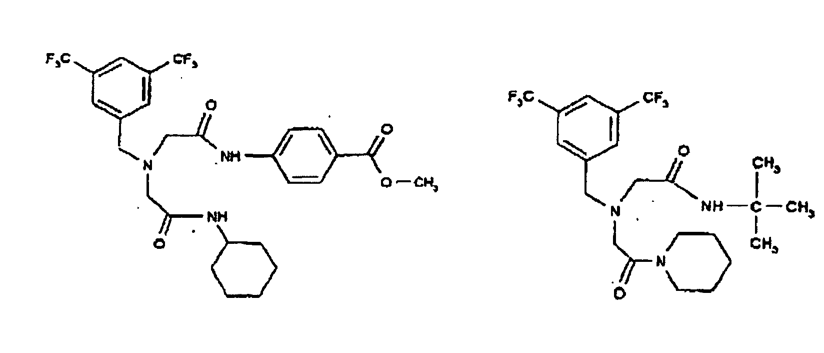

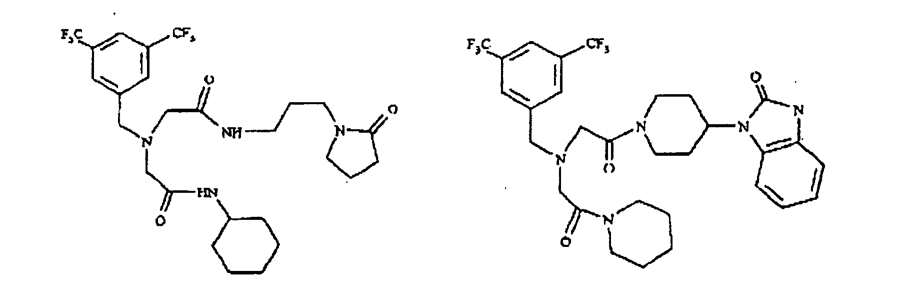

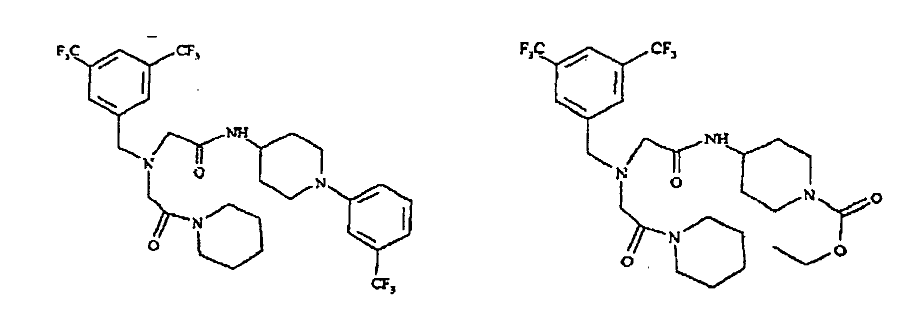

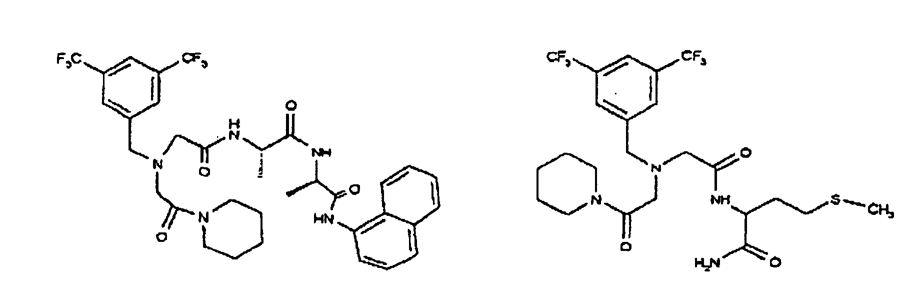

4. Composition according to one of the preceding claims, in which the compound of formula (I) is present in an amount from 0.001 to 20% of the total weight of the composition, in particular in an amount from 0.01 to 10% by weight and preferably in an amount from 0.1 to 5% by weight of the total weight of the composition.

5. Composition according to one of the preceding claims, which is provided in the form of an aqueous, alcoholic, aqueous/alcoholic or oily solution; of a dispersion of the lotion or serum type; of emulsions with a liquid or semi-liquid consistency of the milk type, obtained by dispersion of a fatty phase in an aqueous phase (O/W) or vice versa (W/O); of suspensions or emulsions with a soft consistency of the aqueous or anhydrous gel or cream type; of microcapsules or microparticles; of vesicular dispersions of ionic and/or non-ionic type; in the form of creams, gels or foams; or in the form of aerosol compositions also comprising a pressurized propellant.

6. Composition according to one of the preceding claims, additionally comprising at least one active agent intended for the prevention and/or the treatment of cutaneous conditions, such as agents which modulate cutaneous pigmentation and/or proliferation and/or differentiation; antibacterials; agents for combating parasites; antifungals; antiviral agents; steroidal anti-inflammatory agents; anaesthetic agents; antipruriginous agents; keratolytic agents; agents for combating free radicals; antiseborrhoeics; antidandruff agents; or antiacne agents.

7. Composition according to one of the preceding claims, additionally comprising at least one product with an irritant effect, such as surfactants (ionic or non-ionic), preservatives, organic solvents or active principles, such as α-hydroxy acids (citric, malic, glycolic, tartaric, mandelic or lactic acid), β-hydroxy acids (salicylic acid and its derivatives), α-keto acids, β-keto acids, retinoids (retinol, retinal or retinoic acid), anthralins (dioxyanthranol), anthranoids, peroxides (in particular benzoyl peroxide), minoxidil, lithium salts, antimetabolites, vitamin D and its derivatives, hair dyes or colorants (para-phenylenediamine and its derivatives or aminophenols), perfuming alcoholic solutions (fragrances, eaux de toilette, aftershaves or deodorants), antiperspirant agents (certain aluminium salts), depilatory or permanent-wave active principles (thiols) or depigmenting active principles (hydroquinone).

8. Composition according to one of the preceding claims, additionally comprising at least one compound which decreases the synthesis, the release and/or the activity of at least one mediator of inflammation.

9. Composition according to one of the preceding claims, which is provided in the form of cleansing, protective, treatment or care creams for the face, for the hands, for the feet, for the large anatomical folds or for the body; of liquid foundations, of make-up removal milks, of protective or care body milks or of antisun milks; of lotions, gels or foams for caring for the skin, such as cleansing lotions, antisun lotions or artificial tanning lotions; bath compositions or deodorizing compositions comprising a bactericidal agent; aftershave gels or lotions; depilatory creams; compositions for combating insect stings; pain-control compositions; compositions for treating certain cutaneous disorders; of solid compositions which make up cleansing bars or soaps; of an aerosol composition also comprising a pressurized propellant; of a composition for hair care and in particular a shampoo, a hair-setting lotion, a treating lotion, a styling gel or cream, a dye composition, optionally in the form of colouring shampoos, hair-structuring lotions or a permanent-wave composition; a lotion or gel for combating hair loss or a shampoo for combating parasites; or of a composition for oral use, for example a toothpaste.

10. Composition according to one of the preceding claims, in which the medium is a cosmetically acceptable medium.

11. Composition according to one of the preceding claims, intended to prevent and/or treat cutaneous disorders and in particular cutaneous irritations, sores, redness, dysaesthetic sensations, warming sensations and/or pruritus of the skin and/or mucous membranes.

12. Composition according to one of the preceding claims, intended to treat sensitive skin.

13. Compound corresponding to the following formula (Ia): 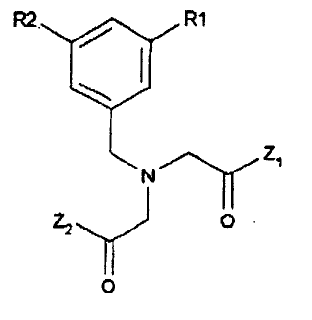 in which:
- R₁ and R₂, which are identical or different, are chosen from hydrogen or CH₃, CF₃, OCH₃ and OH radicals, with the proviso that at least one of the R₁ and R₂ radicals is other than hydrogen;
- Z₁ and Z₂ have the same meaning as in the above Claim 1.

14. Compound according to Claim 13, in which:
- the R₁ and R₂ radicals represent CF₃ and/or
- the Z₁ and Z₂ radicals represent piperidine or methionamide.

15. Compound according to either of Claims 13 and 14, chosen from the compounds corresponding to one of the following formulae:

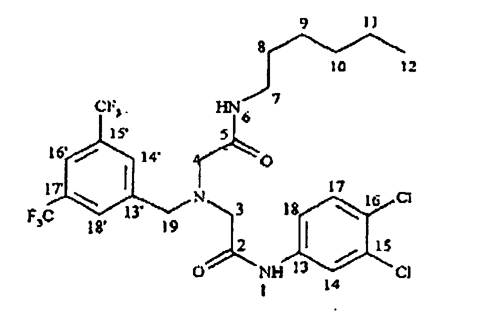

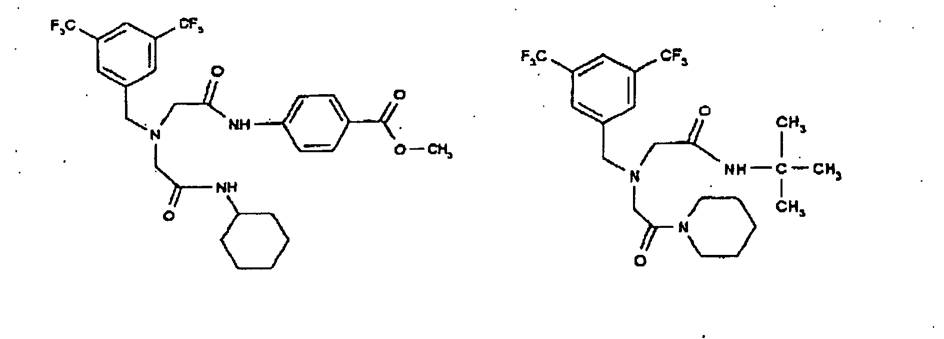

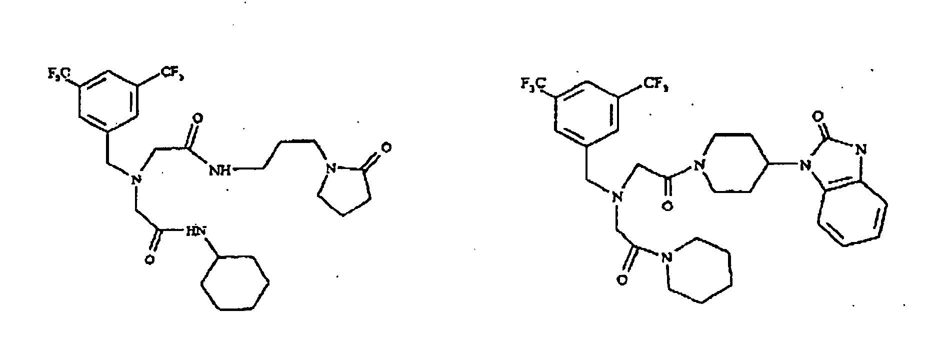

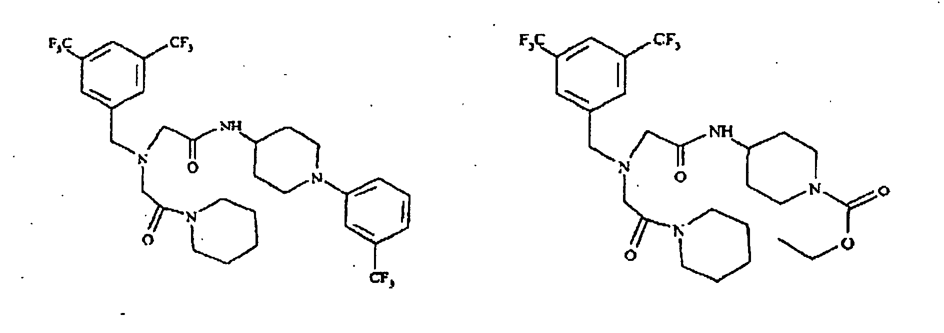

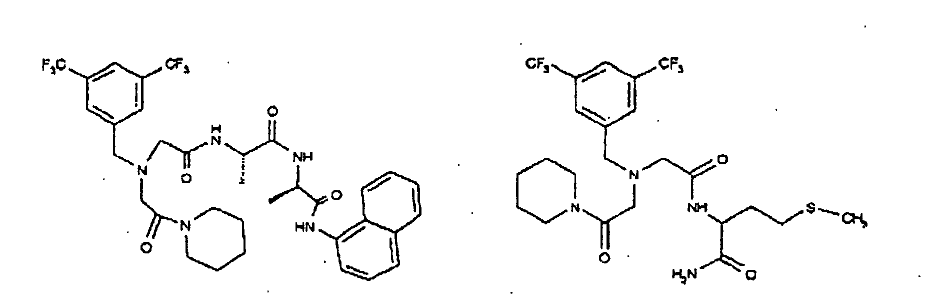

16. Process for the preparation of a compound of formula (Ia) according to Claim 13, in which a diacid is reacted with a dehydrating agent, so as to obtain an intermediate anhydride which makes possible coupling with a first amine or a first alcohol, and then, in a second stage, a coupling agent is added which makes possible coupling of the said diacid with a second amine or a second alcohol.

17. Use of at least one compound of formula (I) as defined in one of Claims 1 to 3, in a cosmetic composition furthermore comprising a product with an irritant effect in order to weaken, indeed even eliminate, the said irritant effect.

18. Use of at least one compound of formula (I) as defined in one of Claims 1 to 3, in a cosmetic composition or for the preparation of a physiologically acceptable composition, in order to prevent and/or treat cutaneous disorders and in particular cutaneous irritations, sores, redness, dysaesthetic sensations, warming sensations and/or pruritus of the skin and/or mucous membranes.

19. Use according to Claim 18, for treating sensitive skins.

20. Cosmetic treatment process, **characterized in that** a cosmetic composition as defined in any one of Claims 1 to 12 is applied to the skin, to the hair and/or to the mucous membranes.
